# EUROPEAN PATENT APPLICATION

(11) **EP 2 402 454 A1**
(43) Date of publication of application: **04.01.2012**
(21) Application number: 10167946.2
(22) Date of filing: 30.06.2010
(51) Int. Cl.: C12P 19/12

(54) **Cellobiose production from biomass**

(71) Applicant: Süd-Chemie AG, 80333 München (DE)
(72) Inventor: Kohl, Andreas Dr., 81369 München (DE); Brück, Thomas Dr., 82067 Ebenhausen (DE); Gerlach, Jochen, 80687 München (DE); Zavrel, Michael Dr., 81379 München (DE); Röcher, Lutz, 81541 München (DE); Kraus, Michael Dr., 82178 Puchheim (DE)
(74) Representative: HOFFMANN EITLE

(57) **Abstract**

The invention is directed to a method for the enzymatic production of cellobiose from sugar beet and other cellulose-containing biomass. Cellobiose is a disaccharide which consists of two molecules of glucose connected via a β-1,4 linkage. The process of the current invention allows for economic production of cellobiose and high yields.

The process for producing cellobiose according to the invention comprises the following steps: subjecting a cellulose containing material to a cellulase treatment at a dry matter content between 7.5 and 50 % (w/w), preferably between 10 and 25% (w/w), and more preferably between 12 and 20% (w/w), followed by separating the cellobiose containing liquid phase from the solid phase.

## Description

### Technical Field

The invention is directed to a method for the enzymatic production of cellobiose from sugar beet and other cellulose-containing biomass. Cellobiose is a disaccharide which consists of two molecules of glucose connected via a β-1,4 linkage. The process of the current invention allows for economic production of cellobiose and high yields.

### Technical Background And Prior Art

Cellobiose is a valuable ingredient in the food, animal feed, chemicals, cosmetics and pharmaceutical industries. Cellobiose can be produced by chemical and biochemical methods from polymeric cellulose or from disaccharides such as sucrose.

When using cellulosic feedstocks, cellobiose can be prepared either by chemical acidolysis or by enzymatic hydrolysis. During chemical hydrolysis, cellulose is subjected to acids, such as sulfuric acid or glacial acetic acid to produce cellobiose octaacetate which is subsequently converted to cellobiose by chemical deacetylation (Pereira at al., (1988): Methods Enzymol., 160, 26). During enzymatic hydrolysis, cellobiose is produced from a cellulose containing feedstock by treatment with cellulose-hydrolyzing enzymes (Taniguchi (1989, Nippon Nogeikagaku Kaishi, 63, 1133). The enzymatic method can lead to higher yields and in contrast to the chemical methodology does not require toxic reagents nor is it associated with waste disposal problems.

When wood pulp or paper pulp are utilised for the enzyme catalysed cellobiose production, a physico-chemical pretreatment, which may encompass a hydrothermal treatment in the absence or presence of a base or acid catalyst, such sulfuric acid (US 4908311, JP2006204294) can be applied. These physicochemical pre-treatments reduce the crystallinity index of the substrate, which increases the propensity for cellulase enzyme attack on the cellulose portion of the substrate, thus enabling higher product yields (JP2006204294). However, pre-treatments at high temperatures (> 120 °C) and/or high acid concentrations (> 1 % w/v) result in the formation of inhibitory compounds that reduce enzyme hydrolysis activity and limit product yields (Palmqvist and Hahn-Hägerdal (2000), Bioresource Technology, 74, pp. 25-33).

In addition to physicochemical methods, mechanical (i.e. washing, grinding, sieving, drying) and enzymatic pretreatments can be applied to remove unwanted biomass components, such as pectins and hemicelluloses (Bonnin, E. et al. (2000) Carb.Poly., 41, pp. 143-151, Micard, V. et al., (1997) Lebensmitt. Wiss. U. Technol. 30, pp. 284-291) from complex biomass such as sugar beet pulp. In these processes pectinase preparations with very low cellulose side-activities are applied to selectively solubilise and remove non-cellulose components. Subsequently, the remaining cellulose enriched solid phase can be contacted with cellulase enzyme preparations to obtain cellobiose.

Commercial cellulase preparations useful for cellobiose production are complex mixtures of hydrolysing enzyme activities. These activities can be grouped into endocellulases (e.g. endoglucanases), exo-cellulases (e.g. cellobiohydrolases) and di- and oligosaccharide hydrolizing enzymes (e.g. β-glucosidases, also often referred to as cellobiase). While endo- and exo-cellulases release oligo-and disaccharides (cellobiose) from polymeric cellulose chains, β-glucosidases (beta-glucosidases, BGL) convert these products into glucose monomers. Hence, for the optimal production of cellobiose, BGL activities are undesirable. Most cellulase preparations contain β-glucosidase activity, which will degrade cellobiose to glucose. This β-glucosidase activity can be inhibited by the addition of an inhibitor such as D-gluconolactone (Reese et al., 1971, Carbohydrate Research, 18, 381-388).

While inhibition of BGL activities can be accomplished *in-*situ by addition of inhibitors, such as D-glucono-lactone (Bonnin et al., (2000), Carbohydr. Polymers 41, pp. 143-151), removal of BGL from the enzyme complex is commonly achieved by chromatographic means. Native or processed-cellulose (Tanaka, M. (2000), The Bull.of Okayama Univer. Of Sci. 36, pp. 217-223;Hommaet al. (1992), J. Chem. Engineer. of Japan 25, pp. 288-93) or chitin (Hommaet al., (1993), Biotechnol. and Bioengineer. 41, pp. 405-410) can be used as chromatographic resins which allow selective retention of endo- and exo-cellulase enzymes.

Glucose oxidase has been applied for the removal of glucose from egg white (Sisak et al., 2006, Enzyme and Microbial Technology, 39, 1002-1007), to remove glucose from a mixture with mannose (Mislovicova et al., 2009, Journal of Molecular Catalysis B: Enzymatic, 60, 45-49) and from dextran oligosaccharides (Mislovicova et al., 2009, Process Biochemistry, 42, 704-709). For the purpose of glucose oxidation either molecular oxygen or several oxidizing agents such as artificial ruthenium or osmium complexes are required (Ivanova et al., 2003, Biochem. Moscow, 68(4), 407-415; Wohlfahrt et al., 2004, Mol. Cell. Biochem., 260, 69-83).

According to the prior art, cellobiose is released from cellulosic substrates by adding a cellulase enzyme at an enzyme to substrate ratio of 1 - 20 % w/w dry matter at 45 - 55 °C for 2 - 600 hours in a 50 - 200 mM, pH: 4.5 - 5.5 buffer (Micard et al., (1997), Lebensm.-Wiss.u.-Technol. 30, pp. 284-291, Micard et al. 2000, Vanderghemet al. (2009), Appl. Biochem. Biotechnol. DOI 10.1007/s12010-009-8724-7) .

The cellobiose yield is limited by the catalytic efficiency of cellobiohydrolase (CBH) which is significantly inhibited by the end product cellobiose (Ki - 0,3 mM). Process options have been described to increase cellobiose yield that remove a cellobiose containing liquid phase is removed in timely intervals from the reaction mixture. The process has been described to run in batch, fed-batch or continuous mode (Micardet al., (1997), Lebensm.-Wiss.u.-Technol. 30, pp. 284-291, Bonnin et al.(2000), Polymers 41, pp. 143-151, Vanderghem et al. (2009), Appl.Biochem.Biotechnol. DOI 10.1007/s12010-009-8724-7, Homma et al., (1993) , Biotechnol.and Bioengineer. 41, pp. 405-410, ; Tanaka, M. (2000), The Bull. of Okayama Univer. of Sci. 36, pp. 217-223). Cellobiose yields between 2 and 40% w/w with regard to the initial cellulose into the desired product have been described. The cellobiose selectivity of the enzymatic hydrolysis process has been described to be between 60 - 85% w/w (Micardet al., (1997), Lebensm.-Wiss. u. -Technol. 30, pp. 284-291; WO 9203565).

In order to purify cellobiose, the by-product glucose needs to be separated from the cellobiose besides other impurities. Glucose can be removed by passing the solution through an immobilized yeast column whereby glucose is metabolized selectively by the yeast. Cellobiose is recovered afterwards (US 4,908,311).

Cellobiose can also be recovered by cooling the solution to 0 °C or below to precipitate cellobiose. Alternatively, an organic solvent can be added to precipitate the cellobiose. Desalting and decoloring of cellobiose can be done by fractionation on a strongly acidic cation-exchanger followed by repeated crystallisation.

Known methods for the enzymatic production of cellobiose from cellulose utilise raw material streams (e.g. paper pulping waste waters) which contain a low cellulose content (2 - 7 % w/v dry matter). Consequently, the cellobiose obtained from the enzymatic reaction is at relatively low concentrations. The comparatively large containers needed for the enzymatic degradation of any given cellulose amount and the optional need to concentrate the obtained cellobiose solution are inefficient from an economic point of view.

Known methods for the enzymatic production of cellobiose from cellulose also require high enzyme dosing regimes of up to 10 % enzyme/substrate w/w. Therefore, a reduction of enzyme needed is desirable from an economic point of view.

The cellobiose can be used as a building block in polymers, as food ingredient, such as animal feed additive, for example as pre-biotic additive, as cosmetics additive or ingredient, or in an anti-inflammatory pharmaceutical preparations (Briton et al. (2003) Ann.New York.Acad.Sci. pp. 750-54; Briton et al. (2003) Hemijsk Industrija 57, pp. 622-625, US 5219842; Kurita et al., (2003), Poly.Chem.Ed. 18, pp. 365-370).

### PROBLEM TO BE SOLVED

Enzyme costs and cellobiose product yields are critical parameters of enzymatic cellobiose production methods from an economic point of view.

The problem to be solved by the present invention is therefore the provision of an economic method for production of cellobiose, comprising an enzymatic degradation of cellulose from a cellulose-containing material. The problem to be solved by the present invention also includes the provision of a method for producing cellobiose at high concentrations.

### SUMMARY OF THE INVENTION:

The present invention provides an economically efficient process for producing cellobiose from a cellulose containing material, whereby high substrate yields can be achieved. The present invention also provides an economically efficient process for producing cellobiose from a cellulose containing material, whereby low enzyme/substrate are used.

In particular, the present invention covers a process for producing cellobiose, comprising the following steps:
c) subjecting a cellulose containing material to a cellulase treatment at a dry matter content between 7.5 and 50 % (w/w), preferably between 10 and 25% (w/w), and more preferably between 12 and 20% (w/w), and
d) separating the cellobiose containing liquid phase from the solid phase obtained in c).

The substrate used for the production of cellobiose is a cellulose containing material. Many cellulose containing materials are suitable substrates for the process according to the present invention, in particular microcrystalline cellulose, bleached and unbleached pulp from the paper industry, organocell pulp, paper, paperboard, cardboard, wood, woodchips, bark, wheat and other grain straw, corn stover, sugar cane bagasse, sugar beet pulp, potato peel, vegetable residues, or fruit pulp. In a preferred embodiment, the cellulose containing material used as substrate in the process of the present invention is or comprises sugar beet pulp.

In a more preferred embodiment, the process above is preceeded by the follwong steps: subjecting a cellulose containing material to a treatment that produces a liquid phase containing pectin, hemicellulose and/or their degradation products, and a solid phase containing cellulose; and separating the solid phase obtained is separated from the liquid phase and subjected to the above-mentioned cellulase treatment.

In a preferred embodiment, the enzymatic treatment above is carried out in the presence of an enzyme preparation comprising at least one type of polypeptide exhibiting cellobiohydrolase activity.

It is preferred that the cellulase composition used exhibits reduced or minimal β-glucosidase activity. Various ways for obtraining such a celulase composition are possible, including, but by no means limited to: obtaining the cellulase composition from an organism devoid of enzymes exhibiting β-glucosidase activity, removing any polypeptides exhibiting β-glucosidase activity, inhibiting any polypeptides exhibiting β-glucosidase activity.

In a more preferred embodiment, the cellobiose cellobiose containing liquid phase obtained by the process above is subjected to a further work-up step, which comprises: optionally concentrating the cellobiose solution, and further purifying the cellobiose from the (optionally concentrated) cellobiose solution. The cellobiose may be purified for example by one or more of the following: cellobiose crystallization, chromatography, nanofiltration, precipitation by organic solvents, or the use of microorganisms, whereby crystallization is preferred.

It is possible to carry out the removal of cellobiose containing liquid phase subsequently to the removal of cellobiose containing liquid phase. However, in an alternative embodiment, it is also possible to carry out the removal of cellobiose containing liquid phase in parallel to the removal of cellobiose containing liquid phase.

It is also possible in the sense of the present invention to remove any glucose that may be present in the cellobiose containing liquid phase. A preferred way of glucose removal is its oxidation to gluconic acid. Gluconic acid may then be removed by one or several of the following: ion exchange chromatography, precipitation, electrodialysis, whereby ion exchange chromatography is preferred.

### DETAILED DESCRIPTION OF THE INVENTION

The present invention provides an economically efficient process for producing cellobiose from a cellulose containing material, whereby high substrate yields can be achieved. The present invention also provides an economically efficient process for producing cellobiose from a cellulose containing material, whereby low enzyme/substrate are used.

### Substrate:

The substrate used for the production of cellobiose is a cellulose containing material. Many cellulose containing materials are suitable substrates for the process according to the present invention, in particular microcrystalline cellulose, bleached and unbleached pulp from the paper industry, organocell pulp, paper, paperboard, cardboard, wood, woodchips, bark, wheat and other grain straw, corn stover, sugar cane bagasse, sugar beet pulp, potato peel, vegetable residues, or fruit pulp. In a preferred embodiment of the invention the relevant substrate contains a significantly larger ratio of amorphous cellulose versus crystalline cellulose. The cellulose containing material which is subjected to the enzymatic hydrolysis step described below preferably contains amorphous cellulose at 50 % or more relative to the total amount of cellulose, and which is or comprises preferably fruit pulp or vegetable residue, or is sugar beet biomass or is or comprises even more preferably sugar beet pulp. Preferred is a content of 50 % or more amorphous cellulose relative to the total amount of cellulose (w/w), and particularly preferred is a content of 60 - 99% amorphous cellulose (w/w) in the cellulose containing material subjected to the enzymatic hydrolysis step.

In another preferred embodiment the cellulose containing substrate is treated by physicochemical means prior to enzymatic hydrolysis. Physicochemical treatment can be acid treatment or heat treatment or any combination thereof such that part or the entire crystalline cellulose portion of the substrate is rendered amorphous.

In a preferred embodiment the cellulose containing substrate is any form of sugar beet, vegetable or fruit biomass. In a particularly preferred embodiment the substrate is sugar beet pulp, or vegetable residues, or fruit pulp residues. In an especially preferred embodiment, the cellulose containing material used as substrate in the process of the present invention is or comprises sugar beet pulp. "Sugar beet pulp" means the residual biomass material after extraction of part or all sucrose from sugar beet (Beta *vulgaris).* Sugar beet pulp can be used freshly after extraction, with or without pressing. Fresh sugar beet pulp typically has a dry matter content between 19 and 29 % w/w. Sugar beet pulp can also be used after drying, with or without pelleting. Dried sugar beet pulp typically has a dry matter content of 85 - 90 % w/w.

The substrate can be cellulosic or lignocellolosic material. The material can either be fresh (e.g. fresh sugar beet pulp, wet cellulose from a paper mil), or dried (e.g. dried straw, dried sugar beet pulp, wood, paper) or in any other form, including any form of preserved materials. In a particularly preferred embodiment the substrate is fresh sugar beet pulp, ensiled sugar beet pulp, dried sugar beet pulp, or dried and pelleted sugar beet pulp.

In a preferred embodiment sugar beet pulp is the substrate and is treated with acid at elevated temperature at high dry matter content. The transportation of such material into and out of the pretreatment reactor is achieved by using a conveyer belt or a screw conveyor. The mixing can be carried out by using special kinds of stirring equipment such as anchor agitators or segmented spiral stirrers, which guarantee a uniform power input as well as a homogeneous distribution of temperature and added substances.

**Hydrolysis of the cellulose comprised in the substrate to cellobiose:**

The present invention covers a process for producing cellobiose, comprising the following steps:
c) subjecting a cellulose containing material to a cellulase treatment at a dry matter content between 7.5 and 50 % (w/w), preferably between 10 and 25% (w/w), and more preferably between 12 and 20% (w/w), and
d) separating the cellobiose containing liquid phase from the solid phase obtained in c).

The hydrolysis can be performed in various ways, e.g. as batch, repeated batch, continuous, semi-continuous, using a membrane based process, or in a counter-flow setup, or in any combination thereof. That means, in a more preferred embodiment, the steps c) and d) of the process above are repeated multiple times, whereby fresh aqueous phase, and optionally no cellulase or no enzyme preparation comprising a polypeptide exhibiting cellulase activity is added to the solid phase in subsequent cycles. The enzyme composition and various preferred embodiments thereof are given in detail below.

In a more preferred embodiment, steps c) and d) of the process above are carried out simultaneously, by continuously replacing the cellobiose containing liquid phase by fresh aqueous phase.

In a preferred embodiment, a repeated batch process is used, dry matter content between 7.5 and 50 % (w/w), preferably between 10 and 25% (w/w), and more preferably between 12 and 20% (w/w), and an enzyme-to-substrate ratio of 0.05 - 1 % enzyme d.m substrate (w/w). This allows for short cyling times and a high overall yield.

In another preferred embodiment, a membrane reactor is used, which enables the continuous removal of the cellobiose containing liquid phase. The membrane is preferably a combination of a crude membrane, an ultrafiltration membrane with cut-off between 500 and 50 kD, followed by a nanofiltration step. In another preferred embodiment, the ultrafiltration membrane has a cut-off between 3.5 and 30 kD. The nanofiltration step is used to concentrate the cellobiose.

In another preferred embodiment a microfiltration membrane is used besides an ultra- and a nanofiltration membrane in order to improve the clarification of the product stream.

The above mentioned process allows for an efficient recycling of cellulases, an easy recovery of cellobiose. It also allows the usage of a smaller reactor size for the processing of any given amount of cellulose containing material and it allows an efficient re-use of the process water. Each of these advantages, as well as a combination thereof, lead to cellobiose production process with improved economic properties.

### Enzyme composition

The cellulase treatment of step b) above for cellobiose production may be carried out using an enzyme preparation or enzyme composition. This enzyme preparation preferably comprises at least one polypeptide having cellobiohydrolase activity (EC 3.2.1.91, CBH).

In a preferred embodiment, the enzyme/substrate ratio in step c) of the process above is 0.01 to 0.5 %, preferably 0.05 to 0.1 %.

In a more preferred embodiment of the invention, the cellulase preparation also contains at least one polypeptide with beta-endo-glucanases activity (EC 3.2.1.4, EG) to break down the polymeric substrate cellulose into smaller fragments which are more amendable to hydrolysis by a cellobiohydrolase activity.

In one embodiment of the invention, the cellulase preparation is derived from a fungal fermentation, e.g. from the cultivation supernatant of fungi such as *Trichoderma, Thermoascus, Talaromyces* sp., or *Penicillium* sp. Such cellulase preparations, besides cellobiohydrolases (EC 3.2.1.91, CBH) and endoglucanases (EC 3.2.1.4, EG) often also contain β-(beta-)glucosidases (EC 3.2.1.21, BGL) that hydrolyse cellobiose to glucose.

In a preferred embodiment of the present invention, the cellulase preparation used for degradation of the cellulose containing material in the process above exhibits reduced β-glucosidase activity.

In an even more preferred embodiment, the cellulase preparation used in the process above originates from an organism devoid of enzymes that exhibit significant β-glucosidase activity, and preferably from an organism wherein at least one gene, more preferably all genes encoding polypeptides exhibiting β-glucosidase activity, have been deleted or rendered dysfunctional.

In a preferred embodiment, the original cellulase preparation contains β-glucosidase activity, but the β-glucosidase activity is minimized or eliminated by adding the original cellulases preparationto an adsorption material selected from cellulose-, chitin- and chitosan-containing materials, followed by washing the adsorption material, which yields a cellulase preparationcontains β-glucosidase activity wherein the β-glucosidase activity is minimized or eliminated. In an especially preferred embodiment, the adsorption material is the cellulose-contianing solid phase obtained in step a) of the process described below. Briefly, said step a) comprises subjecting a cellulose containing material to a treatment that produces a liquid phase containing pectin, hemicellulose and/or their degradation products, and a solid phase containing cellulose.

In a more preferred embodiment, β-glucosidase activity is removed or reduced by passing the cellulase preparation over sugar beet pulp, which leads to selective retention of cellulase (CBH, EG) activites on the designated substrate, whereas β-glucosidase activity is removed or reduced, minimized or eliminated.

In another preferred embodiment, a strain is used for the production of the cellulase preparation that is deficient in β-glucosidase activity. The β-glucosidase deficient expression strain can be generated by conventional mutagenesis and selection, or by selective molecular biology and generation of specific knock out strains.

In another preferred embodiment, the enzyme preparation comprising a polypeptide exhibiting cellulase activity in the process above comprises at least one cellobiohydrolase from the following group of cellobiohydrolases (EC 3.2.1.91): a glycosylhydrolase belonging to family 6 or family 7 of glycosylhydrolases. The families of glycosylhydrolases are well known to the person skilled in the art, and have been classified by Henrissat et al. (1998, FEBS Lett. 425(2): 352-354). Especially preferred are polypeptides exhibiting cellobiohydrolase activity that belong to one of the following: Cellobiohydrolase I, Cellobiohydrolase II. Even more preferred are the following polypeptides exhibiting Cellobiohydrolase activity: CBHI from *Trichoderma reesei* (SEQ ID NO. 1) , CBHII from Trichoderma reesei (SEQ ID No. 2), a polypeptide exhibiting cellobiohydrolase activity and having 70 % or more, preferably 80 % or more, and even more preferably 90 % or more identity with any of SEQ ID NO. 1 or SEQ ID NO. 2. It is particularly preferred that the polypeptide exhibiting cellobiohydrolase activity is produced by secretion from a recombinant yeast or fungal expression host that is devoid of measurable β-glucosidase activity.

In a preferred embodiment of the invention, the cellulase preparation is produced from an organism devoid of enzymes that exhibit any measurable β-glucosidase activity. Preferably an organism is used wherein at least one gene, more preferably all genes encoding for polypeptides exhibiting measurable β-glucosidase activity have been deleted or rendered dysfunctional. In a preferred embodiment, a Trichoderma *reesei* strain is used in which one or more genes encoding polypeptides exhibiting β-glucosidase activity have been deleted or rendered dysfunctional. In a particularly preferred embodiment, a *Trichoderma* reesei strain is used in which at least the gene bgl1 (E.C. 3.2.1.21) has been deleted or rendered dysfunctional.

In another preferred embodiment, a recombinant cellulase or recombinant cellulases are used, which are heterologously expressed by an expression host which does not exhibit any detectable BGL activity. Preferably at least one gene encoding one of the following cellobiohydrolase enzymes is cloned by recombinant DNA techniques into an expression host: a glycosylhydrolase belonging to family 6 or family 7 of glycosylhydrolases (Henrissat et al. 1998, FEBS Lett. 425(2):352-354). Especially preferred are polypeptides exhibiting cellobiohydrolase activity that belong to one of the following: Cellobiohydrolase I, Cellobiohydrolase II. Even more preferred are the following polypeptides exhibiting Cellobiohydrolase activity: CBHI from *Trichoderma* reesei (SEQ ID NO. 1), CBHII from *Trichoderma reesei* (SEQ ID No. 2), a polypeptide exhibiting cellobiohydrolase activity and having 70 % or more, preferably 80 % or more, and even more preferably 90 % or more identity with any of SEQ ID NO. 1 or SEQ ID NO. 2. The cellobiohydrolases are preferably produced by secretion from a recombinant yeast or fungal expression host that is devoid of measurable β-glucosidase activity. A non-limiting list of such organisms includes *Pichia pastoris*, *Saccharomyces cerevisiae, Hansenula polymorpha, Kluyveromyces lactis.*

In another embodiment of the invention, the cellulase preparation contains β-glucosidase activity, but the enzymatic activity of polypeptides with β-glucosidase activity is inhibited by addition of an inhibitor, such as D-gluconolactone. The D-gluconolactone can be added to the reaction or can be produced in situ, from glucose by the enzyme glucose oxidase and oxygen. D-gluconolactone may decompose spontaneously and form gluconic acid (Guiseppi-Elie et al., 2009, Journal of Molecular Catalysis B: Enzymatic, 58, 118-123).

In a preferred embodiment of the invention, D-gluconolactone is continuously formed in-situ and thus it is present during the complete time of reaction despite its possible decomposition. Besides the inhibiting effect, the oxidation of glucose to D-gluconolactone with subsequent spontaneous degradation to gluconic acid facilitates the purification of cellobiose later on by e.g. ion exchange chromatography or electrodialysis.

In another embodiment of the invention, the concentration of the formed by-product hydrogen peroxide, which has a damaging effect on glucose oxidase, is minimized by immobilization of glucose oxidase on activated carbon, which has a strong catalytic action for hydrogen peroxide decomposition. This has the additional advantage that the half amount of the consumed molecular oxygen is recovered.

In a more preferred embodiment of the invention, catalase or activated carbon is added to the reactor. Catalase or activated carbon catalyzes the decay of hydrogen peroxide to water and molecular oxygen. For this reason, it is possible to produce the molecular oxygen also in-situ by feeding hydrogen peroxide to the hydrolysis reactor avoiding complicated ventilation with air or oxygen. In another preferred embodiment, the by-product glucose is removed by the use of glucose oxidase (GOX) from cellobiose produced by alternative enzymatic methods. Alternative methods are the enzymatic production of cellobiose from starch (Masayuki Suzuki, Kyoko Kaneda, Yukiko Nakai, Motomitsu Kitaoka and Hajime Taniguchi, New Biotechnology Volume 26, Issues 3-4, 31 October 2009, Pages 137-142) or from sucrose (US 5077205, 1991) by using phosphorylases. Other alternative methods utilize the transferase activity of the β-glucosidase at high concentrations of glucose (Murray P, Aro N, Collins C, Grassick A, Penttilä M, Saloheimo M, Tuohy M., Protein Expr Purif. 2004 Dec;38(2):248-57).

The enzyme mixture comprising a polypeptide exhibiting cellulase activity can be of different bacterial, plant, yeast or fungal origin, e.g. from bacterial origin such as Bacillus sp., *Streptomyces* sp., Pseudomonas sp., *Escherichia* sp., or from yeast origin, e.g. from *Pichia* sp., *Saccharomyces* sp., Candida sp., *Kluyveromyces* sp., *Yarrowia* sp., *Schizosaccharomyces* sp., or from other fungal origin, e.g. from *Acremonium* sp., *Aspergillus* sp., *Aureobasidium* sp., *Cryptococcus* sp., *Filibasidium* sp., Fusarium sp., *Humicola* sp., *Penecillium* sp., *Talaromyces* sp., *Thermoascus* sp., or *Trichoderma* sp.

In a preferred embodiment the enzyme preparation comprising a polypeptide exhibiting cellulase activity originates from a *Trichoderma* sp., more specifically from *Trichoderma reesei, Trichoderma harzianum, Trichoderma koningii*, *Trichoderma longibrachiatum* or *Trichoderma* viride. In particularly preferred embodiment the above-mentioned cellulase preparationoriginates from *Trichoderma reesei.*

The cellulase preparation is either produced in its native host, e.g. originating from bacteria, plant, yeast or fungi, or the enzymes are expressed heterologously by a host organism. Such recombinant expression host can be a bacterium, a yeast or a fungal strain, e.g. *Escherichia coli,* Bacillus *subtilis, Bacillus brevis,* Bacillus *coagulans, Bacillus megaterium, Bacillus stearothermophilus, Streptomyces* sp., Pseudomonas sp., *Pichia* pastoris, Pichia angusta, *Saccharomyces cerevisiae, Kluyveromyces* sp., Hansenula sp., *Yarrowia sp.,* Trichoderma *reesei, Aspergillus niger, Aspergillus oryzae,* or *Penicillium* sp.

To adjust the substrate dry matter content in the reaction, the dry mass of the respective biomass is determined, preferably by an IR-balance.

To adjust enzyme dosing regimes with respect to substrate dry matter content, the enzyme concentration is determined by measuring total protein content of the respective enzyme stock solution using the colorimetic Bradford (Bradford, M. M. (1976), I Anal. Biochem. 72, pp. 248-254) method. Protein concentrations are determined with respect to a linear standard curve, which has been obtained with bovine serum albumin as a calibration reference. The solid, cellulose containing substrate, optionally after solid-liquid separation, is incubated with the cellulase preparation at one or more of the following conditions: constant temperature, constant pH, constant pressure, dry substance content and enzyme-substrate ratio.

Thus, in an even more preferred embodiment the cellulosic material is incubated with the cellulase preparation for 0.1 - 24 hours at 10 - 80 °C and a pH between 3.0 and 9.0 and an enzyme-to-substrate ratio of 0.01 - 3 % w / w d.m. substrate. The protein content of the cellulase preparation and the dry matter content of the substrate are used to adjust the enzyme-to-substrate w/w ratio.

In a more preferred embodiment the cellulose containing material is incubated with the cellulase preparation for 0.5 - 6 hours, at 40 - 60 °C at a pH between 4.5 and 5.5 with a dry matter content of 2 - 20 % and an enzyme-to-substrate ratio of 0.05 - 1 % w/w d.m substrate.

In an even more preferred embodiment, the cellulose containing material is incubated with the cellulase preparation for 2 - 6 hours at 45 - 55 °C at a pH between 4.5 - 5.0 with a dry matter (d.m.) content of 7.5 - 15 o and an enzyme-to-substrate ratio of 0.05 - 0.1 % w/w d.m. substrate.

### Substrate treatment before hydrolysis:

It is preferred in the process according to the present invention, that the substrate is further treated before enzymatic hydrolysis. The aim of the treatment is to provide better availability of the cellulose in the subsequent hydrolysis step.

The treatment can be a chemical, physical, or enzymatic processing, or a combination thereof. The treatment can be either of the following, an acid treatment, by addition of an inorganic or organic acid, a base treatment, by addition of an inorganic or organic base, application of temperature, or pressure, or application of an organosolv process, by addition of an organic solvent, the use of ionic liquids, or a hydrothermal pretreatment, with or without application of pressure, or freeze-thaw cycles, or addition of any catalyst, homogeneous or heterogeneous, or addition of one or more enzymtic activities, or any combination of the above. In a preferred embodiment the treatment combines physical and chemical (i.e. physicochemical) processing. In a particularly preferred embodiment, the substrate is heated in combination with acid treatment.

In a preferred embodiment sugar beet pulp is the substrate and is treated with acid at elevated temperature at high dry matter content. As outlined above, hydrolysis can be performed in various ways, e.g. as batch, repeated batch, continuous, semi-continuous, using a membrane based process, or in a counter-flow setup, I or in any combination thereof.

In a particularly preferred embodiment, the dry matter content is between 7.5 and 50 % w/w. In a more preferred embodiment the dry matter content is between 10 and 25% w/w. In an even more preferred embodiment, I the dry matter content is between 12 and 20% w/w.

In a preferred embodiment the cellulose containing substrate is treated with an acid in combination with temperature and pressure, whereas the acid can be any inorganic or organic acid, the temperature is within the range of 80 - 210 °C and the pressure is between 0.5 and 100 bar. In more preferred embodiment the temperature is between 95 and 135 °C.

In another preferred embodiment, the cellulose containing substrate is treated with an acid in combination with temperature and pressure, whereby the acid is either sulfuric acid (H₂SO₄), , sulfurous acid (H₂SO₃), hydrochloric acid (HC1), carbonic acid (H₂CO₃) or carbon dioxide (CO₂), nitric acid (HNO₃), or nitrous acid (HNO₂). The temperature used in combination with acid is within the range of 80 - 150 °C and the applied pressure is within 1 - 10 bar. In embodiment, the temperature is within the range of 80 - 210 °C and the applied pressure is within 0.5 - 100 bar. In a more preferred embodiment the temperature is between 95 and 135 °C.

In a particularly preferred embodiment sulfuric acid is added to the substrate and the substrate is treated at a dry matter content between 20 and 25 % w/w at a temperature between 95 and 135 °C, The sulfuric acid dosage is preferably between 0.1 and 1% w/w (sulfuric acid/substrate).

The treatment renders part or all of the non-cellulose material in the substrate soluble. Thereby a portion of the non-cellulose material after the treatment is in the liquid phase whereas the majority of the cellulose is in the solid phase.

### Separation of non-cellulose material from the substrate:

In one embodiment of the invention part or all of the soluble non-cellulose material is removed before the cellobiose-producing hydrolysis step.
Non-cellulose material can comprise hemicellulose, pectin, hydrolysis products from hemicellulose and pectin, lignin, mineral salts, organic acids, alcohols, I oligosaccharides, sucrose, and/or other soluble sugars.

Thus, the present invention also covers a process for producing cellobiose, comprising the following steps:
a) subjecting a cellulose containing material to a treatment that produces a liquid phase containing pectin, hemicellulose and/or their degradation products, I and a solid phase containing cellulose;
b) separating the solid phase obtained in a) from the liquid phase;
c) subjecting the solid phase obtained in b) to a cellulase treatment, wherein the dry matter content of the solid phase obtained in a) is 7.5 to 25 % (w/w), preferably 10 to 20% (w/w), thereby providing a cellobiose containing liquid phase and a solid phase;
d) separating the cellobiose containing liquid phase from the solid phase obtained in c).

In a preferred embodiment, I the dry matter content during the treatment in step a) is between 7.5 and 50 % (w/w), preferably between 10 and 30 %.

It has been found by the authors of the present invention that, when the substrate is sugar beet pulp and the substrate is treated with acid at a dry matter content between 20 and 25 % w/w at a temperature between 95 and 135 °C, a majority of the non-cellulose material is rendered soluble whereas the majority of the cellulose remains unsoluble. The non-cellulose material of sugar beet pulp comprises pectin (polygalacturonic acid) and hemicellulose (galactoarabinan), comprising the monomers galacturonic acid, arabinose, galactose, rhamnose and/or xylose. Typically at least 80 % w/w of the pectin and hemicellulose fraction is renedered soluble by the described treatment whereas at least 80 % w/w of the cellulose remains in the solid phase.

The portion of the substrate that is rendered soluble by the treatment step is removed by solid-liquid-separation. The solid-liquid separation can be performed by continuous or discontinuous centrifugation, filter pressing, or band pressing methods.

In another preferred embodiment pectinases and arabinases are added prior to the separation step. Preferably, incubation with these enzymes is carried out for a duration of 10 min to 12 hours at a temperature between 20 and 60 °C. In a more preferred embodiment, I pectinases and arabinases are added prior to the separation step, and incubated for a duration of 10 min to 4 hours at temperatures between 30 and 50 °C.

Preferably the pectinase and arabinase enzyme is produced by culturing one of the following microorganisms: *Aspergillus niger, Penicillium foetidus.*

In a particularly preferred embodiment, the liquid phase after separation is used as a source for value adding products. It contains polymers like pectin, arabinan, galactan, rhamnogalacturonan, xylan, as well as oligomeric forms and monomers from these polymers. The polymers can be used in purified form or as a mixture (e.g. as thickener, surfactant, emulsifier, in feed or food applications). In addition, the liquid phase contains monomeric sugars and sugar derivatives of the above mentioned polmers in various amounts (0-100% total sugar and sugar acid contents), e.g. galacturonic acid, galactose, arabinose, xylose, rhamnose, glucose and fructose.

The monomeric sugars and sugar acids can be used directly, after purification as pure substances, or in various mixtures. They can be used as source for value adding products, after chemical or biochemical conversion to yield sugar acids (e.g. gluconic acid, galactonic acid, arabonic acid, xylonic acid, rhamnonic acid), sugar diacids (e.g. galactaric acid, glucaric acid, arabaric acid, xylaric acid, rhamnaric acid), sugar alcohols (e.g. xylitol, arabitol, galactitol, rhamnitol), furfural, hydroxymethylfurfual, furandicarboxylic acid and deriviatives thereof. The conversion step is done by enzymatic treatment, by homogeneous and/or by heterogeneous catalysis.

The removal of the liquid phase after the treatment step is beneficial for the process since it improves the efficiency of the enzymatic hydrolysis of the cellulose by cellulases, e.g. by removing inhibiting substances. Thus, in a preferred embodiment, the present invention relates to a process for producing cellobiose from a cellulose containing material, wherein the liquid phase is removed after the hydrolysis step.

### Cellobiose work-up and purification:

The cellobiose containing liquid phase obtained by the process above can be collected and concentrated.

In another preferred embodiment, the cellobiose contained in the cellobiose containing liquid phase of step d) of the process above is subjected to a further step e), which comprises: optionally concentrating the cellobiose solution, and further purifying the cellobiose from the (optionally concentrated) cellobiose solution, preferably to a purity of at least 50 %, more preferably of at least 60 *%* more preferably of at least 70 %, more preferably of at least 80 %, even more preferably of at least 90% (w/w), and most preferably of at least 99% (w/w).. In an even more preferred embodiment, step e) comprises one or more of the following: cellobiose crystallization, chromatography, nanofiltration, precipitation by organic solvents, or the use of microorganisms, whereby crystallization is preferred.

Concentration can by achieved by processes well known in the art, e.g. by evaporating water, by membrane techniques, or by removing water via extraction into another liquid phase.

In a preferred embodiment, the water is removed by a membrane technique using ultra-, micro- and nano-filtration, concentrating the cellobiose from a concentration between 0.1 % and 1.5 % (w/w) up to a concentration of up to 20 % (w/w). This can be followed by a further concentration step using a thin film evaporizer, to concentrate the cellobiose from 10% (w/w) up to 400 (w/w).

In a more preferred embodiment, the water is removed by a membrane technique using ultra-, micro- and nano-filtration, concentrating the cellobiose from a concentration between 0.1 % and 1.5 % (w/w) up to 10% (w/w), followed by a further concentration step, using a thin film evaporizer to concentrate the cellobiose from 10% (w/w) up to 25% (w/w). In another preferred embodiment the cellobiose containing liquid is concentrated by a thin film evaporizer, concentrating the cellobiose from 0.1% up to 40% (w/w).

In a more preferred embodiment the cellobiose containing liquid is concentrated by a thin film evaporizer, concentrating the cellobiose from 0.1% up to 25% (w/w).

The concentrated liquid contains 50 - 95 % w/w cellobiose (relative to the total amount of saccharides), 1 - 40 % w/w glucose, and minor quantities of e.g. arabinose, xylose, galactose, rhamnose, galacturonic acid, fructose and other impurities.

It is possible to further purify the cellobiose by e.g. crystallization, chromatography, nanofiltration, precipitation by organic solvents, or the use of microorganisms, whereby crystallization is preferred.

The resulting liquid can have a brownish color. Therefore, optionally a decoloration step is added to the process which removes part or all of the colored substances. This is preferably done by adsorption of the impurities to an adsorber material, preferably to charcoal, I ion exchange resins or bentonites. In a preferred embodiment, this is done by a chromatography step using an adsorption to an adsorption material which is or comprises bentonite.

### Process for purifying a cellobiose from a composition comprising cellulose and glucose

The present invention also comprises a process for purifying cellobiose from a composition comprising cellobiose and glucose, comprising a step of converting glucose to gluconic acid by glucose oxidation in the presence of glucose oxidase (GOX) and oxygen.

In this process, the by-product glucose is removed from the cellobiose by the use of glucose oxidase (GOX) (and optionally catalase or activated carbon) to oxidize the glucose to gluconic acid. Catalase or activated carbon is added for the purpose to remove the product H₂O₂ and to avoid unspecific oxidation of the product cellobiose.

Thus, glucose in the liquid phase containing cellobiose obtained by the process above is converted to gluconic acid by glucose oxidation, preferably in the presence of glucose oxidase (GOX) and oxygen. In an even more preferred embodiment, the gluconic acid obtained by the process above is removed from the liquid phase containing cellobiose by one or several of the following: ion exchange chromatography, precipitation, electrodialysis, whereby ion exchange chromatography is preferred.

In one embodiment, gluconic acid may be removed from the reaction mixture by ion exchange chromatography.

In an alternative embodiment, the glucose present in the liquid phase may be converted to D-gluconolactone *in situ.* The D-gluconalactone may then be used to inhibit the activitiy of any polypeptides exhibiting β-glucosidase activity.

The process for obtaining cellobiose according to the present invention may be carried out in such a way, that the enzymatic activity of any polypeptides with P-glucosidase activity is inhibited by an inhibitor, preferably D-gluconolactone, whereby the D-gluconolactone is preferably generated in situ from glucose comprised in the liquid phase, according to the process for glucose oxidation described above.

Many other monosaccharides can also be oxidized by GOX, even though the specific activity is much lower, and subsequently be removed.

In a preferred embodiment, immobilized, thermostable glucose oxidase, I catalase or activated carbon and oxygen are used to oxidize glucose to gluconic acid, at 30 - 60 °C, and substrate concentrations of 5 - 50 g/l glucose and other monosaccharides. Afterwards, the gluconic acid is removed by standard ion exchange chromatography. The gluconic acid can be recovered from the ion exchange resin, e.g. by elution with an acid. In another preferred embodiment, gluconic acid is removed by precipitation, or by electrodialysis making use of an anion-selective membrane.

To improve the purity of the cellobiose, it is possible to use microorganisms (e.g. bacteria, yeast, fungi), which can process glucose, other monomeric sugars and sugar acids, but are unable to break the beta-glycosidic bond in cellobiose and are therefore unable to digest cellobiose.

In a preferred embodiment, the used microorganisms are yeasts (e.g. *Saccharomyces cerevisiae, Klyveromyces* sp., *Schizosaccharomyces* sp.). In an even more preferred embodiment *Saccharomyces cerevisiae* is used for this process step.

Another option for purification of cellobiose involves selective crystallization of the cellobiose, , in order to purify the product and to produce an easily storable product. The method for final preparation and storage of the product is not restricted to any particular one, and can involve crystallization, spray-drying, drum-drying, extruder techniques and simple evaporation of water in a concentration process. The product can be used as amorphous powder, in a crystalline form or as a cellobiose syrup.

In an even more preferred embodiment, the cellobiose obtained by the process above is further purified to a purity of more than 99% (w/w).

### Use of the cellobiose preparation obtainable by the process according to the present invention:

The cellobiose preparation obtainable by the process according to the present invention can be applied as food ingredient, animal feed additive, cosmetics ingredient. The cellobiose preparation can be used in an anti-inflammatory pharmaceutical preparation.

The produced cellobiose can be used in many applications, e.g. as pre-biotic substance for feed and food applications, as a low caloric ingredient in food, as a buildung block in polymers, in organic synthesis, as additive in polymers, as an active pharmaceutical ingredient in therapeutic applications, I as building block in polymers, or in cosmetic applications.

In a one embodiment the cellobiose is used as a feed additive that improves the animal feed product. The feed can be used for feeding pigs, piglets, calves, cattle, and/or poultry such as chicken and turkey. The cellobiose is typically used in a concentration of 0.01 - 1 % w/w of cellobiose in the dry matter content of the animal feed composition.

In a preferred variant of this embodiment, the cellobiose is used in a concentration of 0.05 - 0,5 %w/w of cellobiose. In a more preferred variant of this embodiment the cellobiose is used in a concentration of 0.05 - 0.2 % w/w of cellobiose.

In another embodiment the combination product containing 50 - 90 % cellobiose (w/w) and 5 - 50 % gluconic acid (w/w) is used as an animal feed ingredient, with a concentration of 0.05 - 1.0 % w/w, more preferably 0.05 - 0.5 % w/w, and even more preferably 0.05 - 0.2 % w/w of cellobiose.

Preferably, the cellobiose preparation is added to an animal feed preparation at a dosage of 0.01 - 1% (w/w), preferably at a dosage of 0.05 - 0.2% (w/w) . The cellobiose preparation can have a prebiotic effect on the intestinal flora of the animal. The addition of cellobiose can improve the overall health status of the animal. Furthermore, I the addition can lead to an increased feed uptake and/or weight gain. The animal feed is preferably used as a feed for pig, fish, cattle and/or poultry, more preferably for pig and poultry.

In another embodiment of the invention, the cellobiose preparation is used as a food ingredient. Applications in food comprise ingredients in baking, beverages, as well as chocolates, I candy and confectionery. In baking and confectionery applications the cellobiose serves as an ingredient that has a low caloric value and low sweetness, but improves the aroma of the food product, e.g. through the formation of Maillard products.

In a preferred embodiment the cellobiose preparation is added to a cake or biscuit dough recipe in combination with sweeteners such as xylitol, maltitol, and/or stevia, and wherein the sucrose dosage in the dough is reduced. In an especially preferred embodiment, the addition of the cellobiose leads to comparable dough characteristics as if the sucrose dosage were not reduced. Such dough characteristics are one or more of the follwing: browning, aroma formation, mouth feel.

In another preferred embodiment the invention relates to the cellobiose preparation described abovefor use in a food composition providing a prebiotic effect to the food thereby improving the overall health status of the person consuming the food, in particular by improving the intestinal flora.

In another embodiment of the invention, the cellobiose preparation is further purified to a purity of more than 99% (w/w). This cellobiose copmposition is intended for use in an oral pharmaceutical composition effective in the treatment of inflammatory bowel diseases, such as Colitis ulcerosa, Crohn's disease, Collagenous colitis, Lymphocytic colitis, Ischaemic colitis, I Diversion colitis and/or Behçet's syndrome.

The gluconic acid obtainable by the process according to this invention may be applied, e.g. in the food or feed industry, as preservative, as cosmetic additive, in organic syntheses.

In another preferred embodiment, the mixture comprising gluconic acid and cellobiose obtainable by the process according to this invention is used as a combination product in the feed industry, combining the beneficial effects of cellobiose with the benefits of an organic acid.

### Best mode of carrying out the invention

The present invention comprises process for producing cellobiose, which may be carried out comprising the following steps:
a) subjecting a cellulose containing material to a treatment that produces a liquid phase containing pectin, hemicellulose and/or their degradation products, and a solid phase containing cellulose;
b) separating the solid phase obtained in a) from the liquid phase;
c) subjecting the solid phase obtained in b) to a cellulase treatment, wherein the dry matter content of the solid phase obtained in b) is 7.5 to 25 % (w/w), preferably 10 to 20% (w/w), r thereby providing a cellobiose containing liquid phase and a solid phase;
d) separating the cellobiose containing liquid phase from the solid phase obtained in c).

The treatment a) can be a chemical, physical, or enzymatic processing, or a combination thereof. The treatment renders part or all of the non-cellulose material in the substrate soluble. Thereby a portion of the non-cellulose material after the treatment is in the liquid phase whereas the majority of the cellulose is in the solid phase.

The separation step b) can be carried out by any method suitable for solid-liquid separation, such as sieving, filtration etc.

The authors of the present invention have found that, the enzymatic hydrolysis step c) can be carried out efficiently when the dry matter content of the solid phase obtained in b) is 7.5 to 25 % (w/w), preferably 10 to 20% (w/w).

The cellulase treatment of step c) can be a treatment with a single type of polypeptide or an enzyme composition comprising at least one type of cellulase. Enzymes or enzyme compositions comprising Cellobiohydrolase activity are preferred.

The enzyme or enzyme composition used in step c) may be obtained from a native or recombinant source.

Preferably, the enzyme or enzyme composition of step c) exhibits reduced or minimal β-glucosidase activity. Methods for obtaining such an enzyme (composition) comprise obtaining it from an organism that exhibits minimal β-glucosidase activity, inhibiting any measurable β-glucosidase activity by addition of inhibitors, such as such as D-gluconolactone, or removing any polypeptides exhibiting measurable β-glucosidase activity by passing the enzyme composition over an adsorption material selected from cellulose-, chitin- and chitosan-containing materials. Typically any polypeptides exhibiting measurable β-glucosidase activity do not bind strongly to such materials and can therefore be eluted, while the desired enzyme(s) remain bound to the adsorption material. It is preferred that the adsorption material used in this step is the cellulose-containing material to be hydrolyzed. Especially preferred is that the adsorption material is the cellulose containing solid phase obtained in step a) below. Typically, the adsorption process is much quicker than the hydrolysis process.

The cellobiose-containing liquid phase may be separated from the solid phase by any method suitable for solid-liquid separation, such as any type of filtration, preferably nan-ultra- or microfiltration. The cellobiose containing liquid phase may be substituted by fresh or recycled aqueous phase.

It is possible to remove the cellobiose-containing liquid phase simultaneously to addition of new aqueous phase, but it is alternatively possible to carry out these steps sequentially.

The present invention also relates to a process for removal of glucose from an aqueous phase comprising the use of glucose oxidase (GOX) (and optionally catalase or activated carbon) in the presence of oxygen to oxidize the glucose to D-gluconolactone. Catalase is necessary to remove the product H₂O₂ and to avoid unspecific oxidation of the product cellobiose. Gluconic acid originating from hydrolysis of D-gluconolactone may be removed from the reaction mixture by precipitation or ion exchange chromatography. D-gluconolactone may be formed in situ; the D-gluconolactone may then be used as an inhibitor of any polypeptides exhibiting measurable (β-glucosidase activity, as described above.

It is also possible to optionally concentrate the obtained cellobiose containing liquid phase and to further purify the cellobiose from the (optionally concentrated) liquid phase, for example by crystallization.

The product obtained by the process of this invention is also an inherent component of the invention.

The product may be used for example as food additive, e.g. as pre-biotic food additive.

The invention also relates to a product for use in the treatment of diseases such as inflammatory diseases.

### DEFINITIONS

The term "sugar beet biomass" refers to the complete and unprocessed root tissue of Beta vulgaris including the outer peel and the internal pulp. Dry tissue typically contains 80% (w/w) soluble sucrose, while beet pulp is mainly composed of polymeric sugars and pectin including typically 7 % pectin (mainly polygalacturan), typically 7 % cellulose and typically 7 % hemicellulose,mainly in the form of araban.

The term "pectin rich biomass" refers to the unprocessed or processed plant tissues of fruits and vegetables tissue, which are composed of polymeric sugars and pectin, including 1 - 50 % pectin, 1 - 80 % cellulose and 1 - 80 % hemicellulose.

The term "dry matter" (d.m.) refers to the mass to biomass ratio determined after removal of water from fresh tissue, e.g. by using an IR-balance.

The term "polymeric substrate" means substances composed of either a specific monomeric constituent or a limited variety of defined monomeric constituents covalently linked in a linear or partially branched molecular structure. An example is the insoluble fraction of raw lignocellulosic feedstocks, , which can contain significant amounts of such polymeric substrates such as cellulose, xylan, mannan, galactan, arabinoxylan, glucoronoxylan, glucomannan, and xyloglucan.

The term "enzymatic activity" of an enzyme refers to the catalytic activity under appropriate conditions under which the enzyme serves as a protein catalyst. For enzymes capable of degrading polymeric or artificial substrates, "enzymatic activity" is the activity at which the enzyme converts specific polymeric or artificial substrates to specific oligomeric or monomeric products.

The term "cellulase" refers to a polypeptide exhibiting cellulase activity.

The term "cellulase preparation" refers to a cellulase or a composition comprising at least one polypeptide exhibiting cellulase activity. In the context of this description, the term "enzyme preparation" is used synomnymously with "cellulase preparation". The cellulase preparation preferably comprises at least one polypeptide having cellobiohydrolase activity (EC 3.2.1.91, CBH).

The term "cellobiohydrolase" or "CBH" (E.C. 3.2.1.91.) refers to an enzyme, which catalyses the hydrolysis of polymeric cellulose to cellobiose and other beta-D-glucose oligomers. The term "β-glucosidase" or "beta-glucosa.dase" or "BGL" (E.C. 3.2.1.21) refers to an enzyme, I which catalyses the hydrolysis of cellobiose, cellotriose, cellotetraose, and other beta-D-glucose oligomers to the corresponding glucose monomer.

### EXAMPLES

### Example 1: Pretreatment of Sugar Beet Pulp

224.7 g of dried sugar beet pulp (89% w/w dry matter content) was mixed with 535 ml water and 240.4 ml 1% H₂SO₄ and incubated under pressure at 135 °C for 1 hour. The solid and liquid phase were separated by filtration. The solid phase was used for cellobiose production.

### Example 2: Removal of Cellobiose Degrading Enzymatic Activities by Adsorption of Cellulase to the Substrate followed by a Washing Step

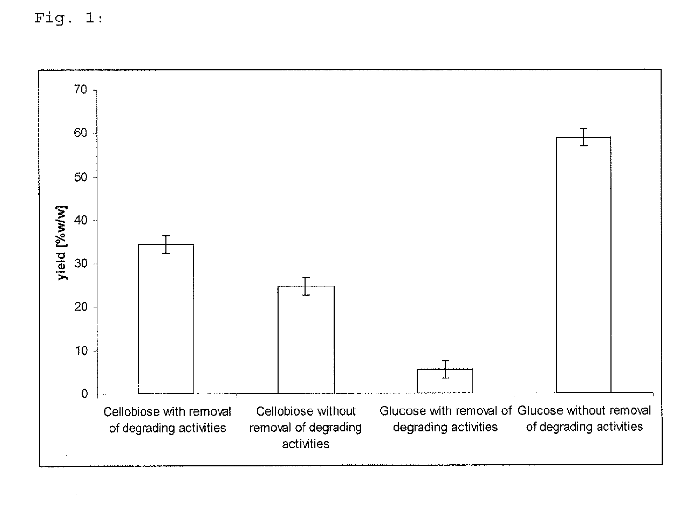

460 g pretreated sugar beet pulp (see Example 1) with a dry matter content of 16,5 % w/w was contacted with 750 mg cellulase preparation from T. reesei for 5 min at room temperature. To remove cellobiose degrading enzymatic activities the liquid phase was removed by filtration. Subsequently 50 mM acetate buffer pH 5.0 was added to adjust the reaction to the same dry mass content (16.5 % w/w). The mixture was incubated for 48 hours at 50 °C followed by a phase separation step. The amount of glucose and cellobiose in the liquid phase was determined by HPLC on a BioRad Aminex HPX 87-H column. Data for the removal of ß-glucosidase activities from cellulose preparation are shown in Fig. 1.

### Example 3: Concentration of cellobiose in liquid phases at Different Dry Matter Factors

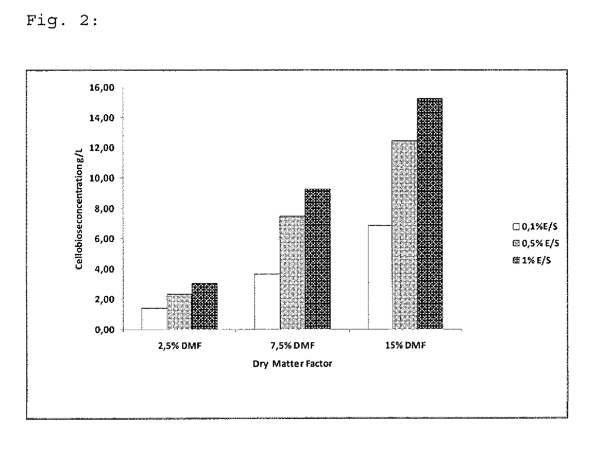

460 g pretreated sugar beet pulp (see Example 1) was contacted with (i) 750 mg, (ii) 375 mg, and (iii) 75 mg cellulase preparation from T. reesei for 5 min at room temperature. Cellobiose degrading activities were removed according the procedure described in example 2. 50 mM acetate buffer pH 5.0 was added to the solid fractions to reach a dry matter content w/w of (i) 15, (ii) 7.5, and (iii) 2.5 %, respectively. The mixtures were incubated for 1 h at 50 °C followed by a phase separation steps. The amount of glucose and cellobiose in the liquid phases was determined by HPLC as described in Example 2. Data for cellobiose concentrations in relation to the substrate dry matter content are shown in Fig. 2.

### Example 4: Cellobiose Production from Sugar Beet Pulp at Different enzyme/substrate (E/S) Ratios

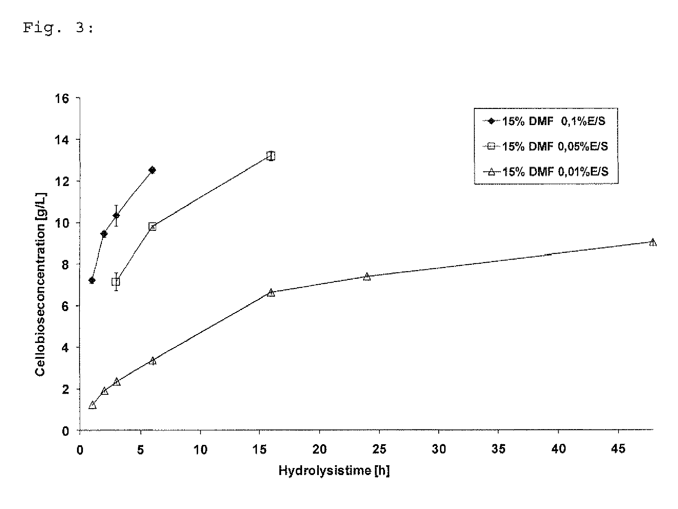

460 g pretreated sugar beet pulp (see Example 1) was contacted with (i) 75 mg, (ii) 37.5 mg, and (iii) 7.5 mg cellulase preparation from T. reesei for 5 min at room temperature. Cellobiose degrading activities were removed according the procedure described in Example 2. 50 mM acetate buffer pH 5.0 was added to the solid fraction to reach a dry matter content w/w of 15 %. The mixtures were incubated at 50 °C and representative samples of the liquid phase were taken at different points of time. The concentration of glucose and cellobiose were determined in the samples by HPLC as described in Example 2. The cellobiose production in relationship to the enzyme substrate ratios are presented in Fig. 3.

### Example 5: Production of Cellobiose from different Cellulosic Materials

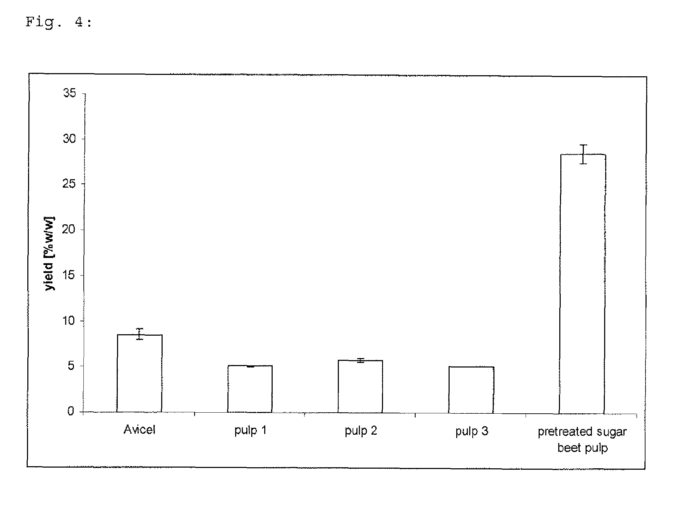

460 g pretreated (see example 1) or untreated sugar beet pulp and Avicel were treated as shown in Example 2 to remove cellobiose degrading activities.
Acetate buffer was added to the solid fraction to reach a dry matter content of 2.5% and the mixture was incubated for 1 h at 50 °C. The amount of glucose and cellobiose in the liquid phases was determined by HPLC as described in Example 2. Cellobiose yields obtained from different cellulosic raw materials are shown in Fig. 4.

### Example 6: Production of Cellobiose from Sugar Beet Pulp

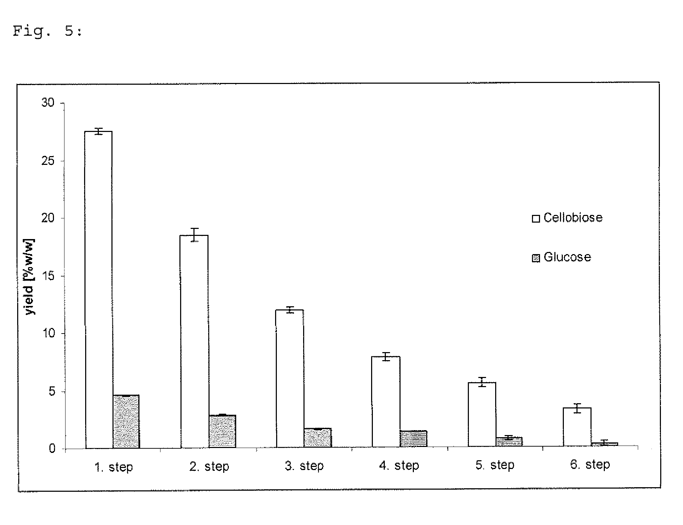

460 g pretreated sugar beet pulp (see Example 1) was treated as shown in Example 2. 50 mM acetate buffer pH 5.0 was added to the solid fraction to reach a dry matter content of 7.5 % w/w. The mixture was incubated for 1 h at 50 °C followed by a liquid/solid phase separation step. The separation of the liquid phase, addition of buffer to the same dry matter content followed by incubation were repeated six times. The amount of glucose and cellobiose were determined in the particular liquid fractions of each step by HPLC as described in Example 2. Glucose and Cellobiose production from sugar beet pulp is shown in Fig. 5.

### Example 7: GOX Substrate specificity

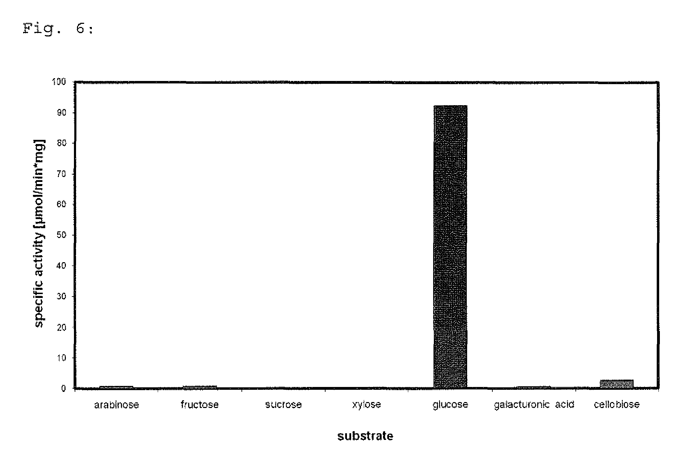

The substrate specificity of glucose oxidase was assessed using the following assay: Each tested saccharide was dissolved in 100 mM Na-acetate buffer at pH 5.5 resulting in a final saccharide concentration of 100 mM. The final solution contained 25 U/mL horseradish peroxidase and 1 mM 2,2'-Azino-bis(3-ethylbenzothiazoline-6-sulfonic acid). The reaction was started by addition of 0.1 U/mL glucose oxidase. The reaction was performed in a shaken 24 well micro-titer plate. The volume in each well was 500 µL. All conditions were tested in triplicates. The absorbance increase was followed at 405 nm. The activity was calculated using a calibration curve with H₂O₂. All reagents were obtained from Sigma-Aldrich (Deisenhofen, Germany) and were of analytical grade. The GOX substrate specificity is shown in Fig. 6.

### Example 8: Conversion of Glucose to Gluconic Acid by Glucose Oxidase

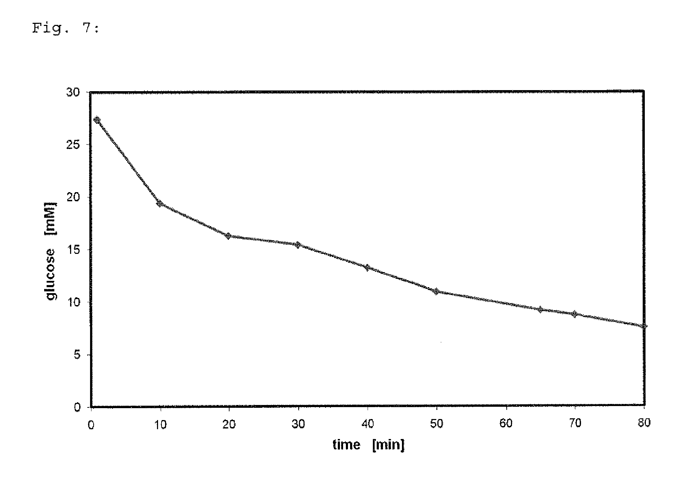

Glucose oxidase was immobilized on glass beads coated with polyethylenimine and glutaraldehyde. 20 mg of these beads with an overall activity of 100 U were added to 100 mL of a 100 mM Na-acetate buffer containing 25 mM glucose, 150 mM cellobiose and 70,000 U catalase. The reaction was performed in a reactor which was continuously vented with air with a ventilation rate of 1 L/min. Aliquots were taken and the glucose concentration was quantified using HPLC. The data for the conversion of glucose to gluconic acid by glucose oxidase are shown in Fig. 7.

### Example 9: Separation of Glucose and Gluconic Acid by Ion Exchange Chromatography

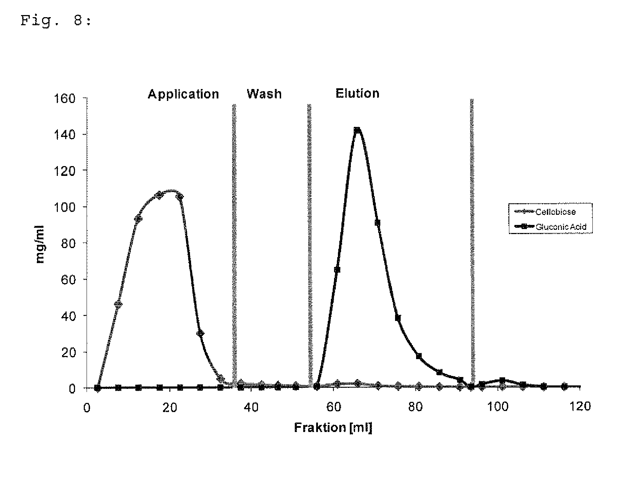

30 ml of the cellobiose / gluconic acid mixture prepared according Example 8 were applied onto 75 ml glass column packed with AMBERLITE IRA67 (Rohm and Haas, Germany). The column was washed with H₂O and eluted with 1M ammonium carbonate. The amount of gluconic acid and cellobiose in the fractions was determined by HPLC as described in Example 2. Data for the HPLC separation of glucose from gluconic acid are shown in Fig. 8.

### BRIEF DESCRIPTION OF THE FIGURES

Fig. 1: Yield of Cellobiose and Glucose in % of Cellulose Substrate in the mixture after Removal of Cellobiose Degrading Enzymatic Activities in the Hydrolysis Step
Fig. 2: Concentration of Cellobiose after Hydrolysis depending on the Dry Matter content
Fig. 3: Cellobiose Production from Sugar Beet Pulp at Different E/S Ratios
Fig. 4: Production of Cellobiose from different Cellulosic Materials
Fig. 5: Yield of Cellobiose after Hydrolysis of Sugar Beet Pulp
Fig. 6: Substrate Specificity Test of Glucose Oxidase
Fig. 7: Decrease of Glucose Concentration due to the Activity of Glucose Oxidase
Fig. 8: Separation of Gluconic acid from Cellobiose by Ion Exchange Chromatography

## Claims

1. A process for producing cellobiose, comprising the following steps:
c) subjecting a cellulose containing material to a cellulase treatment at a dry matter content between 7.5 and 50 % (w/w), preferably between 10 and 25% (w/w), and more preferably between 12 and 20% (w/w), wherein the cellulase treatment is carried out in the presence of an enzyme preparation comprising at least one type of polypeptide exhibiting cellobiohydrolase activity, thereby providing a cellobiose containing liquid phase and a solid phase, and
d) separating the cellobiose containing liquid phase from the solid phase obtained in c).

2. The process for producing cellobiose according to claim 1, comprising the following steps:
a) subjecting a cellulose containing material to a treatment that produces a liquid phase containing pectin, hemicellulose and/or their degradation products, and a solid phase containing cellulose;
b) separating the solid phase obtained in a) from the liquid phase;
c) subjecting the solid phase obtained in b) to a cellulase treatment, wherein the dry matter content of the solid phase obtained in b) is 7.5 to 50 % (w/w), preferably between 10 and 25% (w/w), and more preferably between 12 and 20% (w/w), wherein the cellulase treatment is carried out in the presence of an enzyme preparation comprising at least one type of polypeptide exhibiting cellobiohydrolase activity, thereby providing a cellobiose containing liquid phase and a solid phase;
d) separating the cellobiose containing liquid phase from the solid phase obtained in c).

3. The process according to one or more of the preceding claims, wherein the cellulose containing material which is subjected to step c) contains amorphous cellulose at 50 % or more relative to the total amount of cellulose, and which is or comprises preferably fruit pulp or vegetable residue, or is sugar beet biomass or is or comprises even more preferably sugar beet pulp.

4. The process according to one or more of claims 2 or 3, wherein the dry matter content during the treatment in step a) is between 7.5 and 50 % (w/w), preferably between 10 and 30 %.

5. The process according to one or more of the preceding claims, wherein the enzyme/substrate ratio in step c) is 0.01 to 0.1 %, preferably 0.01 to 0.5 %, and more preferably 0.05 to 0.1 %.

6. The process according to any of the preceding claims, wherein the enzyme preparation originates from an organism devoid of enzymes that exhibit significant β-glucosidase activity, and preferably from an organism wherein at least one gene, more preferably all genes encoding polypeptides exhibiting β-glucosidase activity have been deleted or rendered dysfunctional.

7. The process according to one or more of claims 2 to 5, wherein the cellulase preparation contains β-glucnsidase activity and wherein the cellulase preparation is added to the cellulose containing material to be subjected to step c), followed by washing the material, such that P-glucosidase activity is minimized or eliminated from the solid phase.

8. The process according to one or more of claims 1 to 6, wherein the cellulase preparation comprises at least one polypeptide exhibiting cellobiohydrolase activity from the following group of cellobiohydrolases (EC 3.2.1.91); cellobiohydrolyses of the glycosylhydrolase group GH6, preferably CBHI from *Trichoderma* reesei (SEQ ID NO.1) or cellobiohydrolyses of the glycosylhydrolase group GH7, preferably CBHII from Trichoderma *reesei* (SEQ ID NO. 2), or a polypeptide exhibiting cellobiohydrolase activity and having 70 % or more, preferably 80 % or more, and even more preferably 90 % or more identity with any of SEQ ID NO. 1 or SEQ ID N0. 2, and/or wherein the polypeptide exhibiting cellobiohydrolase activity is preferably produced by secretion from a recombinant yeast or fungal expression host that is devoid of measurable β-glucosidase activity.

9. The process according to one or more of the preceding claims, wherein the cellobiose contained in the cellobiose containing liquid phase of step d) is subjected to a further step e), which comprises: optionally concentrating the cellobiose solution, and further purifying the cellobiose from the (optionally concentrated) cellobiose solution, preferably to a purity of at least 50 %, more preferably of at least 70 %, even more preferably of at least 90% (w/w), and most preferably of at least 99% (w/w).

10. The process according to claim 9, wherein step e) comprises one or more of the following: cellobiose crystallization, chromatography, nanofiltration, microfiltration, ultrafiltration, precipitation by organic solvents, or the use of microorganisms, whereby crystallization is preferred.

11. The process according to one or more of the preceding claims, wherein steps c) and d) are repeated multiple times, whereby fresh aqueous phase is added to the solid phase in subsequent cycles.

12. The process according to one or more of the preceding claims, wherein steps c) and d) are carried out simultaneously, by continuously replacing the cellobiose containing liquid phase by fresh aqueous phase.

13. A process for purifying cellobiose from a composition comprising cellobiose and glucose, comprising a step of converting glucose by glucose oxidation in the presence of glucose oxidase (GOX) and oxygen.

14. The process according to one or more of claims 1 to 12, wherein glucose in the liquid phase containing cellobiose is converted to gluconic acid by the process of claim 14.

15. The process according to claim 14, wherein the gluconic acid obtained by the method of claim 14 is removed from the liquid phase containing cellobiose by one or several of the following: ion exchange chromatography, precipitation, electrodialysis, whereby ion exchange chromatography is preferred.

16. The process according to one or more of claims 1 to 5, wherein the enzymatic activity of any polypeptides with β-glucosidase activity is inhibited by an inhibitor, preferably D-gluconolactone, whereby the D-gluconolactone is preferably generated in situ from glucose comprised in the liquid phase, according to claim 13.

17. Cellobiose preparation obtainable by one or more of claims 1 to 16.

18. Use of a cellobiose preparation of claim 17 as food ingredient, animal feed additive, cosmetics ingredient.

19. Use according to claim 18, wherein the cellobiose preparation is added to an animal feed preparation at a dosage of 0.01 - 1% (w/w), preferably at a dosage of 0.05 - 0.2% (w/w).

20. Use according to claim 18, wherein the cellobiose preparation is added to a cake or biscuit dough recipe in combination with sweeteners such as xylitol, maltitol, and/or stevia, and wherein the sucrose dosage in the dough is reduced.

21. Cellobiose preparation according to claim 17 for use in a method of treating inflammatory bowel diseases, such as Colitis ulcerosa, Crohn's disease, Collagenous colitis, Lymphocytic colitis, Ischaemic colitis, Diversion colitis and/or Behçet's syndrome in a mammal.


SEQUENCE LISTING

<110> Sued-Chemie

<120> Cellobiose production from biomass

<130> HE142572

<160> 2

<170> PatentIn version 3.4

<210> 1
<211> 513
<212> PRT
<213> Trichoderma reesei

<400> 1

Met Tyr Arg Lys Leu Ala Val Ile Ser Ala Phe Leu Ala Thr Ala Arg
1               5                   10                  15

Ala Gln Ser Ala Cys Thr Leu Gln Ser Glu Thr His Pro Pro Leu Thr
            20                  25                  30

Trp Gln Lys Cys Ser Ser Gly Gly Thr Cys Thr Gln Gln Thr Gly Ser
        35                  40                  45

Val Val Ile Asp Ala Asn Trp Arg Trp Thr His Ala Thr Asn Ser Ser
    50                  55                  60

Thr Asn Cys Tyr Asp Gly Asn Thr Trp Ser Ser Thr Leu Cys Pro Asp
65                  70                  75                  80

Asn Glu Thr Cys Ala Lys Asn Cys Cys Leu Asp Gly Ala Ala Tyr Ala
                85                  90                  95

Ser Thr Tyr Gly Val Thr Thr Ser Gly Asn Ser Leu Ser Ile Gly Phe
            100                 105                 110

Val Thr Gln Ser Ala Gln Lys Asn Val Gly Ala Arg Leu Tyr Leu Met
        115                 120                 125

Ala Ser Asp Thr Thr Tyr Gln Glu Phe Thr Leu Leu Gly Asn Glu Phe
    130                 135                 140

Ser Phe Asp Val Asp Val Ser Gln Leu Pro Cys Gly Leu Asn Gly Ala
145                 150                 155                 160

Leu Tyr Phe Val Ser Met Asp Ala Asp Gly Gly Val Ser Lys Tyr Pro
                165                 170                 175

Thr Asn Thr Ala Gly Ala Lys Tyr Gly Thr Gly Tyr Cys Asp Ser Gln
            180                 185                 190

Cys Pro Arg Asp Leu Lys Phe Ile Asn Gly Gln Ala Asn Val Glu Gly

        195                 200                 205

Trp Glu Pro Ser Ser Asn Asn Ala Asn Thr Gly Ile Gly Gly His Gly
    210                 215                 220

Ser Cys Cys Ser Glu Met Asp Ile Trp Glu Ala Asn Ser Ile Ser Glu
225                 230                 235                 240

Ala Leu Thr Pro His Pro Cys Thr Thr Val Gly Gln Glu Ile Cys Glu
                245                 250                 255

Gly Asp Gly Cys Gly Gly Thr Tyr Ser Asp Asn Arg Tyr Gly Gly Thr
            260                 265                 270

Cys Asp Pro Asp Gly Cys Asp Trp Asn Pro Tyr Arg Leu Gly Asn Thr
        275                 280                 285

Ser Phe Tyr Gly Pro Gly Ser Ser Phe Thr Leu Asp Thr Thr Lys Lys
    290                 295                 300

Leu Thr Val Val Thr Gln Phe Glu Thr Ser Gly Ala Ile Asn Arg Tyr
305                 310                 315                 320

Tyr Val Gln Asn Gly Val Thr Phe Gln Gln Pro Asn Ala Glu Leu Gly
                325                 330                 335

Ser Tyr Ser Gly Asn Glu Leu Asn Asp Asp Tyr Cys Thr Ala Glu Glu
            340                 345                 350

Ala Glu Phe Gly Gly Ser Ser Phe Ser Asp Lys Gly Gly Leu Thr Gln
        355                 360                 365

Phe Lys Lys Ala Thr Ser Gly Gly Met Val Leu Val Met Ser Leu Trp
    370                 375                 380

Asp Asp Tyr Tyr Ala Asn Met Leu Trp Leu Asp Ser Thr Tyr Pro Thr
385                 390                 395                 400

Asn Glu Thr Ser Ser Thr Pro Gly Ala Val Arg Gly Ser Cys Ser Thr
                405                 410                 415

Ser Ser Gly Val Pro Ala Gln Val Glu Ser Gln Ser Pro Asn Ala Lys
            420                 425                 430

Val Thr Phe Ser Asn Ile Lys Phe Gly Pro Ile Gly Ser Thr Gly Asn
        435                 440                 445

Pro Ser Gly Gly Asn Pro Pro Gly Gly Asn Arg Gly Thr Thr Thr Thr
    450                 455                 460

Arg Arg Pro Ala Thr Thr Thr Gly Ser Ser Pro Gly Pro Thr Gln Ser

465                 470                 475                 480

His Tyr Gly Gln Cys Gly Gly Ile Gly Tyr Ser Gly Pro Thr Val Cys
                485                 490                 495

Ala Ser Gly Thr Thr Cys Gln Val Leu Asn Pro Tyr Tyr Ser Gln Cys
            500                 505                 510

Leu

<210>  2
<211>  471
<212>  PRT
<213>  Trichoderma reesei

<400>  2

Met Ile Val Gly Ile Leu Thr Thr Leu Ala Thr Leu Ala Thr Leu Ala
1               5                   10                  15

Ala Ser Val Pro Leu Glu Glu Arg Gln Ala Cys Ser Ser Val Trp Gly
            20                  25                  30

Gln Cys Gly Gly Gln Asn Trp Ser Gly Pro Thr Cys Cys Ala Ser Gly
        35                  40                  45

Ser Thr Cys Val Tyr Ser Asn Asp Tyr Tyr Ser Gln Cys Leu Pro Gly
    50                  55                  60

Ala Ala Ser Ser Ser Ser Ser Thr Arg Ala Ala Ser Thr Thr Ser Arg
65                  70                  75                  80

Val Ser Pro Thr Thr Ser Arg Ser Ser Ser Ala Thr Pro Pro Pro Gly
                85                  90                  95

Ser Thr Thr Thr Arg Val Pro Pro Val Gly Ser Gly Thr Ala Thr Tyr
            100                 105                 110

Ser Gly Asn Pro Phe Val Gly Val Thr Pro Trp Ala Asn Ala Tyr Tyr
        115                 120                 125

Ala Ser Glu Val Ser Ser Leu Ala Ile Pro Ser Leu Thr Gly Ala Met
    130                 135                 140

Ala Thr Ala Ala Ala Ala Val Ala Lys Val Pro Ser Phe Met Trp Leu
145                 150                 155                 160

Asp Thr Leu Asp Lys Thr Pro Leu Met Glu Gln Thr Leu Ala Asp Ile
                165                 170                 175

Arg Thr Ala Asn Lys Asn Gly Gly Asn Tyr Ala Gly Gln Phe Val Val
            180                 185                 190

Tyr Asp Leu Pro Asp Arg Asp Cys Ala Ala Leu Ala Ser Asn Gly Glu
        195                 200                 205

Tyr Ser Ile Ala Asp Gly Gly Val Ala Lys Tyr Lys Asn Tyr Ile Asp
    210                 215                 220

Thr Ile Arg Gln Ile Val Val Glu Tyr Ser Asp Ile Arg Thr Leu Leu
225                 230                 235                 240

Val Ile Glu Pro Asp Ser Leu Ala Asn Leu Val Thr Asn Leu Gly Thr
                245                 250                 255

Pro Lys Cys Ala Asn Ala Gln Ser Ala Tyr Leu Glu Cys Ile Asn Tyr
            260                 265                 270

Ala Val Thr Gln Leu Asn Leu Pro Asn Val Ala Met Tyr Leu Asp Ala
        275                 280                 285

Gly His Ala Gly Trp Leu Gly Trp Pro Ala Asn Gln Asp Pro Ala Ala
    290                 295                 300

Gln Leu Phe Ala Asn Val Tyr Lys Asn Ala Ser Ser Pro Arg Ala Leu
305                 310                 315                 320

Arg Gly Leu Ala Thr Asn Val Ala Asn Tyr Asn Gly Trp Asn Ile Thr
                325                 330                 335

Ser Pro Pro Ser Tyr Thr Gln Gly Asn Ala Val Tyr Asn Glu Lys Leu
            340                 345                 350

Tyr Ile His Ala Ile Gly Pro Leu Leu Ala Asn His Gly Trp Ser Asn
        355                 360                 365

Ala Phe Phe Ile Thr Asp Gln Gly Arg Ser Gly Lys Gln Pro Thr Gly
    370                 375                 380

Gln Gln Gln Trp Gly Asp Trp Cys Asn Val Ile Gly Thr Gly Phe Gly
385                 390                 395                 400

Ile Arg Pro Ser Ala Asn Thr Gly Asp Ser Leu Leu Asp Ser Phe Val
                405                 410                 415

Trp Val Lys Pro Gly Gly Glu Cys Asp Gly Thr Ser Asp Ser Ser Ala
            420                 425                 430

Pro Arg Phe Asp Ser His Cys Ala Leu Pro Asp Ala Leu Gln Pro Ala
        435                 440                 445

Pro Gln Ala Gly Ala Trp Phe Gln Ala Tyr Phe Val Gln Leu Leu Thr
    450                 455                 460

Asn Ala Asn Pro Ser Phe Leu
465                 470